# EUROPEAN PATENT SPECIFICATION

(11) **EP 0 315 720 B1**
(45) Date of publication and mention of the grant of the patent: **15.09.1993**
(21) Application number: 87310025.9
(22) Date of filing: 12.11.1987
(51) Int. Cl.: C07H 17/08, A61K 31/70

(54) **4"-Deoxy-3"-demethoxy-3"-methylene-desmycosin derivatives**
4"-Desoxy-3"-demethoxy-3"-methylen-desmycosin-Derivate
Dérivés de 4"-désoxy-3"-déméthoxy-3"-méthylène-desmycosine

(43) Date of publication of application: 17.05.1989
(73) Proprietor: ASAHI KASEI KOGYO KABUSHIKI KAISHA, Osaka (JP)
(72) Inventor: Fujiwara,Tatsuro, Tagata-gun, Shizuoka-ken (JP); Sakakibara, Hideo, Shizuoka-ken (JP); Yashiro,Tomoko, Tagata-gun, Shizuoka-ken (JP)
(74) Representative: Woods, Geoffrey Corlett

(56) References cited:
- FR-A- 2 501 693
- FR-A- 2 541 288
- GB-A- 2 102 793
- JOURNAL OF THE CHEMICAL SOCIETY, PERKIN TRANSACTIONS I, no. 6, June 1987, pages 1189-1229, Royal Society of Chemistry; A.G. FISHMAN et al.: "Novel semisynthetic oxo and alkyl macrolide antibacterials and related derivatives"
- CHEMICAL ABSTRACTS, vol. 107, no. 19, 9th November 1987, page 765, abstract no. 176412c, Columbus, Ohio, US; & JP-A-62 111 995 (SANRAKU CO., LTD) 22-05-1987
- CHEMICAL ABSTRACTS, vol. 105, no. 19, 10th November 1986, page 795, abstract no. 172991b, Columbus, Ohio, US; & JP-A-61 129 195 (TOYO JOZO CO., LTD) 17-06-1986

## Description

This invention relates to macrolide antibiotics, their preparation and pharmaceutical compositions containing them.

Synthetic 16-membered macrolide antibiotics having a mycinose moiety, such as tylosin, are well known. Among them, modification of the mycinose moiety mainly occurs at position-4, for example 4"-deoxy- or 4',4"-di-deoxy-desmycosin derivatives (JP-A-57-154197), 3-O-acyl-4"-deoxy-desmycosin derivatives (JP-A-59-141596), 4"-deoxy- or 4',4"-di-deoxy-19-deformyldesmycosin derivatives (JP-A-58-13596 and 58-121299) and 20-deoxo-20-acylmethylene-4"-deoxy-desmycosin derivatives (JP-A-61-129195).

We have now prepared desmycosin derivatives modified at the position-3". We have found that the 19-deformyl desmycosin derivatives of formula (1) hereinbelow have superior antimicrobial activities as compared with desmycosin. Accordingly, the present invention provides compounds of the formula (1): 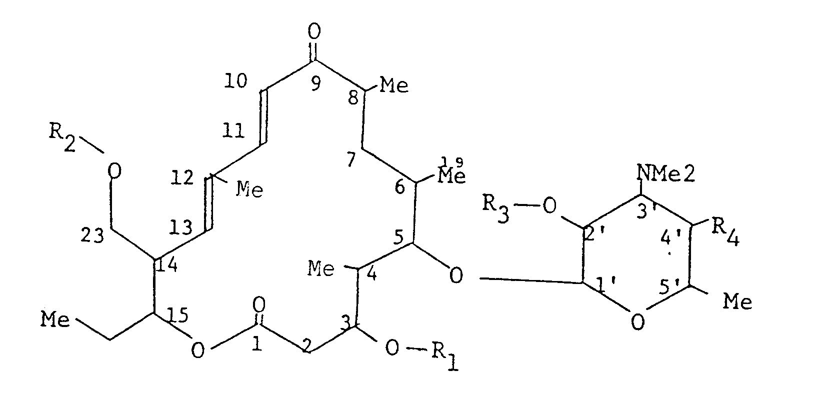
wherein R₁ is hydrogen, C₂-C₅ alkanoyl, unsubstituted benzoyl, unsubstituted phenyl-C₂-C₅ alkanoyl, thienylacetyl or a group of the formula: 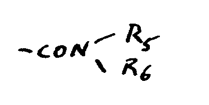
wherein R₅ is C₁-C₄ alkyl, unsubstituted phenyl or unsubstituted phenyl-C₁-C₄ alkyl and R₆ is hydrogen or C₁-C₄ alkyl; R₂ is a 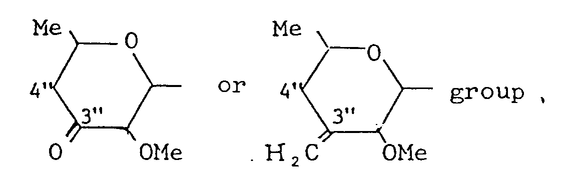
wherein R₃ is hydrogen or protective group for hydroxyl; and R₄ is hydrogen, hydroxyl or protected hydroxyl; and pharmacologically acceptable salts thereof. Me is CH₃.

Preferred compounds are ones in which R₃ is hydrogen and R₄ is hydroxyl. Other preferred compounds are ones in which R₃ or R₄ is hydrogen.

Salts are pharmacologically acceptable salts. Examples of salts are inorganic salts such as the hydrochloride, sulfate or phosphate, and organic salts such as the acetate, propionate, tartrate, citrate, succinate, malate, aspartate or glutamate. Other non-toxic salts can be included.

Desmycosin derivatives of formula (1) and pharmacologically acceptable salts thereof are prepared by a process comprising:
(i) effecting 3",4"-dehydroxylation of the mycinose moiety of 19-deformyl- or 19-deformyl-4'-deoxy-desmycosin wherein the 2'- and, in the case of 19-deformyl-desmycosin, 4'-hydroxy groups are protected to obtain thereby a desmycosin derivative of formula (1'): 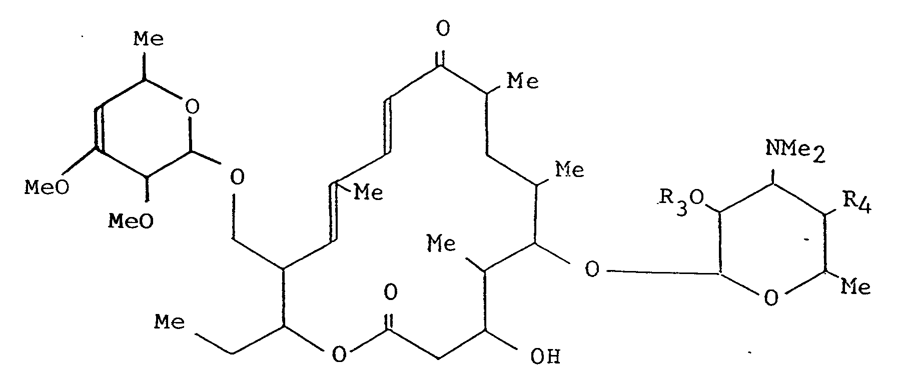 wherein R₃ is a hydroxy-protecting group and R₄ is hydrogen or a protected hydroxy group;
(ii) converting the said desmycosin derivative of formula (1') into a desmycosin derivative of formula (1);
(iii) optionally, removing the or each hydroxy-protecting group; and
(iv) optionally, converting the desmycosin derivative of formula (1) into a pharmacologically acceptable salt thereof.

Step (i) is typically effected by treating a said protected 19-deformyl- or 19-deformyl-4'-deoxy-desmycosin bearing a trifluoromethanesulfonyl substituent at the 4"-position with tetrabutylammonium fluoride.

The production of the compounds (1) of the present invention is illustrated hereinbelow. In this process the formula except for the substituents at positions-3, -2' and 4' and for the mycinose moiety, i.e. the formula 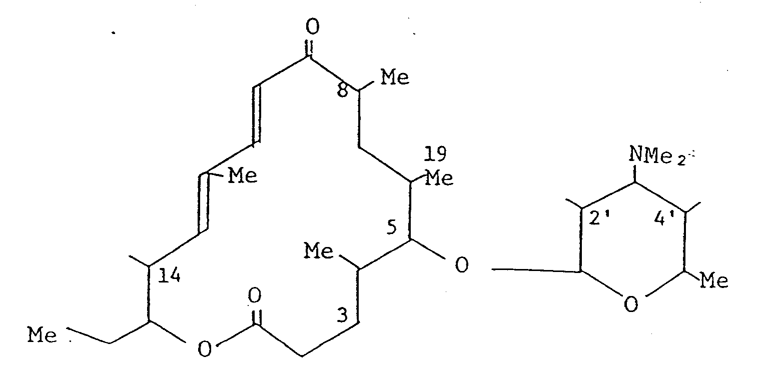
is outlined as 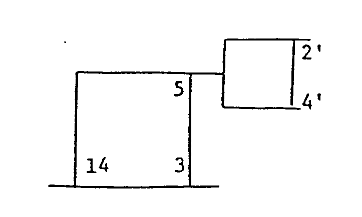
wherein a number in the figure shows position of the substituent.

A compound (1) of the present invention can be prepared, for example, according to the reaction scheme hereinbelow, from 19-deformyl desmycosin or 19-deformyl-4'-deoxydesmycosin. In the production of the compound (1), i.e. (1c)∼(1f) and (1i) or (1l), the production process can be traced by silica-gel thin layer chromatography (TLC) or high performance liquid chromatography (HPLC) and the reaction can be terminated by checking maximum production of the compound.

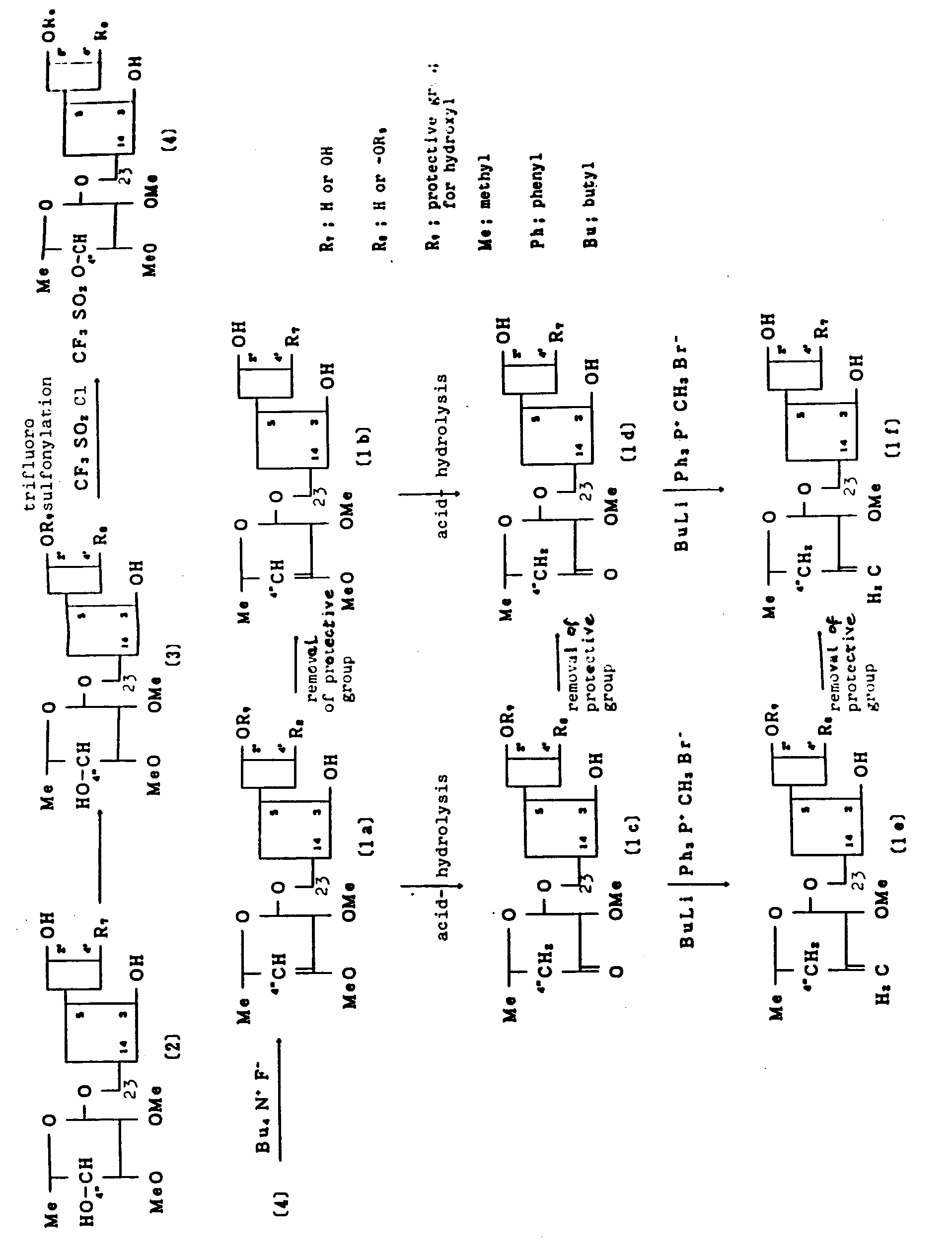

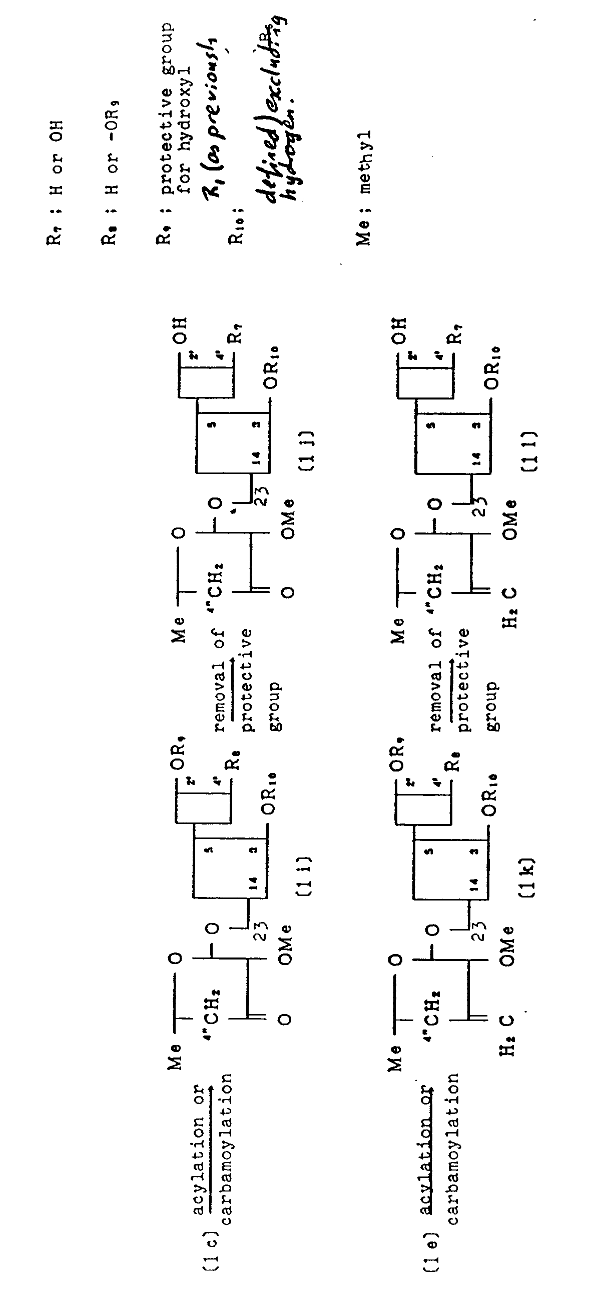

The starting compound (2) is known. 19-deformyldesmycosin is disclosed in JP-A-56-55399 and 19-deformyl-4'-deoxydesmycosin is disclosed in JP-A-58-13596. The hydroxyl group at positions-2' and -4', i.e. when R₇ at position-4' is hydroxyl, in compound (2) is protected before starting the reaction and removed thereafter.

A protective group which can easily removed after the end of the reaction is, for example, lower alkanoyl such as acetyl, propionyl or butyryl or halogenated acetyl such as chloroacetyl, dichloroacetyl, trichloroacetyl or trifluoroacetyl. Acetyl is preferred.

Introduction of an acetyl group can be performed by reacting compound (2) with acetic anhydride in an inert organic solvent. Examples of an inert organic solvent are dichloromethane, chloroform, dichloroethane, acetone and acetonitrile. The preferred reaction temperature is room temperature or below. Isolation of the product (3) can be by a known method such as the process disclosed in JP-A-58-13596 and JP-A-58- 121299.

A method for obtaining a product (4) by trifluoromethane sulfonylation of the hydroxyl at position-4" in compound (3) is, for example, disclosed in JP-A-58-13596 and JP-A-58- 121299.

3",4"-dehydroxylation of mycinose in compound (4) can be carried out by reaction with tetrabutylammonium fluoride in an organic solvent. A preferred organic solvent is tetrahydrofuran. The tetrabutylammonium fluoride is preferably used in an equimolar amount or in slight excess. The reaction proceeds at room temperature or at a slightly elevated temperature. Isolation of the product (1a) can be made by distilling off the reaction solvent, pouring into an aqueous alkali such as aqueous ammonia, extracting with a water immiscible organic solvent such as chloroform and removing the solvent.

Removal of the protective group for R₉, i.e. the protective group for hydroxyl at position-2' and -4', or the protective group for hydroxyl at position-2', in product (1a) can easily be carried out by a known process. For example acetyl can be removed by heating in methanol to obtain the product (1b).

The 3"-demethoxy-3"oxo-compound can be made by hydrolysing the compound (1a) under acidic conditions, for example in an aqueous solvent which may contain a water miscible organic solvent such as acetonitrile, in the presence of an acid such as hydrochloric acid or trifluoroacetic acid. The reaction proceeds under ice-cooling. Isolation of the product (1c) can be made by adjusting the pH of the reaction mixture to 9 to 10 by adding aqueous alkali and extracting with a water immiscible organic solvent such as chloroform.

In the above reaction the compound (1a) can be replaced by a compound (1b), and the product (1d) is consequently obtained.

The 3"-methylene compound (1e) can be prepared by reacting compound (1c) with a methylene triphenylphosphoran solution in an inert organic solvent such as tetrahydrofuran.

The methylene triphenylphosphoran solution is made by pouring methyltriphenylphosphonium bromide (Ph₃P⁺ CH₃Br ⁻ ) into dry tetrahydrofuran and adding butyl lithium (BuLi) dissolved in an organic solvent such as hexane under a stream of an inert gas such as argon.

The reaction of compound (1c) with methylene triphenylphosphoran can proceed under ice-cooling. The methylene triphenylphosphoran is used in molar excess relative to the amount of compound (1c), generally in 3 to 4 times molar excess. 3"-methyleneation preferably proceeds under an atomosphere of an inert gas such as argon. The product (1e) can be isolated from the reaction mixture by adding dilute acid to stop the reaction, adjusting to pH 9 to 10 with an aqueous alkali such as ammonia and extracting with a water immiscible organic solvent such as chloroform.

To prepare the 3"-methylene compound, the compound (1c) can be replaced by a compound (1d), and the product (1f) is consequently obtained. The protective group R₉ in compound (1e) can be removed by the same removing process as described above for compound (1a), and the product (1f) is obtained.

Acylation or substituted carbamoylation of the hydroxyl at position-3 of compounds (1c) or (1e) produces the products (1i) or (1k). The compound (1c) or (1e) is reacted with a carboxylic acid of formula:

R₁₁-COOH (5)

wherein R₁₁ is C₁-C₄ alkyl, unsubstituted phenyl, unsubstituted phenyl-C₁-C₄ alky or thienylmethylene, or a reactive derivative thereof, as an acylating agent.

The carboxylic acid (5) is a C₂-C₅ fatty acid such as acetic acid, propionic acid, butyric acid, isobutyric acid or valeric acid, unsubstituted benzoic acid, an unsubstituted phenyl-C₂-C₅ fatty acid such as phenylacetic acid, 2-phenylpropionic acid, 3-phenylpropionic acid, 2-phenylbutyric acid or 2-phenylisovaleric acid, or thienylacetic acid.

The reactive derivatives are acylating reagents for hydroxyl groups commonly used in organic chemistry, for example an acid halide such as an acid bromide or acid chloride, an acid anhydride, a mixed anhydride, an active ester or an acid azide.

The carboxylic acid (5) can be used together with a known condensation reagent, for example a diimide such as N,N'-di-cyclohexylcarbodiimide (DCC), N- cyclohexyl-N'-2-(morphoryl-4) - ethylcarbodiimide, N,N'-carbonyl-bis-imidazole or iso-oxazolium salt.

The acylation generally proceeds in an organic solvent. Preferred organic solvents are acetone, tetrahydrofuran, dioxane, dimethylformamide, dimethylacetamide, chloroform, dichloromethane and pyridine. If an acid is generated in the acylating reaction, a tertiary organic amine such as triethylamine, pyridine, picolin, collidine, quinoline, isoquinoline, N-methylpiperadine, N-methylmorpholine, dimethylaniline, dimethylaminopyridine or tribenzylamine is preferably added The acylation proceeds at room temperature or at most under heating to 50 to 60°C.

Isolation of the product (1i) or (1k) wherein R₁₀ is acyl as defined above can be made by adding water to the reaction mixture and extracting with a water immiscible organic solvent such as chloroform, dichloroethane, methylisobutyl ketone, ethyl acetate or butyl acetate under an alkaline pH of 8 to 10.

The protective group R₉ in the compound (1i) or (1k) wherein R₁₀ is acyl is removed by the same process as for the removal of the protective group in the compound (1a), to obtain the compounds (1j) or (1l).

The carbamoylation reaction can be carried out by reacting the product (1c) or (1e) with N,N'-carbonyl-di-imidazole in an inert organic solvent and reacting the thus obtained imidazolide compound, which may optionally be isolated from the reaction mixture, with an amine of the formula: 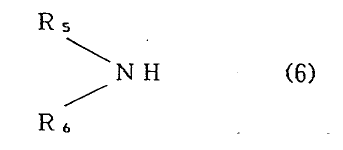
wherein R₅ and R₆ are as defined above.

The imidazolide compound can be isolated by pouring the reaction mixture into water, extracting the aqueous layer with a water immiscible organic solvent such as chloroform under weakly acidic conditions and removing the organic solvent therefrom.

Examples of the amine (6) are mono-C₁-C₄ alkylamines such as methylamine, ethylamine, propylamine or butylamine, di-C₁-C₄ alkylamines such as dimethylamine, diethylamine, dipropylamine, methyl ethylamine, methyl propylamine or ethyl propylamine, unsubstituted phenylamine, N-C₁-C₄ alkyl-phenyl, in which the phenyl is unsubstituted, such as N-methylaniline, unsubstituted phenyl-C₁-C₄ alkylamine such as benzylamine, β-phenylethylamine, α-methylbenzylamine or β-phenylpropylamine, or N-C₁-C₄ alkyl-phenyl-C₁-C₄ alkylamine, in which the phenyl is unsubstituted, such as N-methylbenzylamine.

The above reaction with the imidazolide compound and the amine (6) can be performed in an inert organic solvent under heating. However, when the reaction proceeds at room temperature heating is not required. Examples of inert organic solvents are dichloroethane, benzene, toluene and dioxane.

Isolation of the product (1i) or (1k) wherein R₁₀ is substituted carbamoyl can be made by the same procedure as in the process isolating the compound wherein R₁₀ is acyl.

A protective group R₉ in the compound (1i) or (1k) wherein R₁₀ is substituted carbamoyl is removed by the same process as the removal of the protective group in the compound (1a). Thus the compound (1j) or (1ℓ) wherein R₁₀ is substituted carbamoyl is consequently obtained.

The thus obtained compound (1), i.e. compounds (1c)∼(1f) and (1i)∼(1ℓ), can be purified if required at any process steps or at the final step. For example, the product can be purified by column chromatography using silica-gel, activated alumina or an adsorption resin by eluting with suitable solvent such as benzene-acetone or chloroform-methanol.

The minimum inhibitory concentration (MIC) of a compound (I) of the present invention is illustrated in Table 1.

In the Table 1 the compounds are:
- Compound 1:: 4"-deoxy-3"-dimethoxy-3"-oxo-19-deformyldesmycosin,
- Compound 2:: 4"-deoxy-3"-demethoxy-3"-methylene-19-deformyldesmycosin,
- Compound 3:: 4',4"-di-deoxy-3"-demethoxy-3"-methylene-19-deformyldesmycosin,
- Compound 4:: 4"-deoxy-3"-demethoxy-3"-methylene-3-O-propionyl-19-deformyldesmycosin,
- Compound 5:: 4"-deoxy-3"-dimethoxy-3"-methylene-3-O-dimethylcarbamoyl-19-deformyldesmycosin,
- Des:: Desmycosin (4'-demycarosyltylosin).

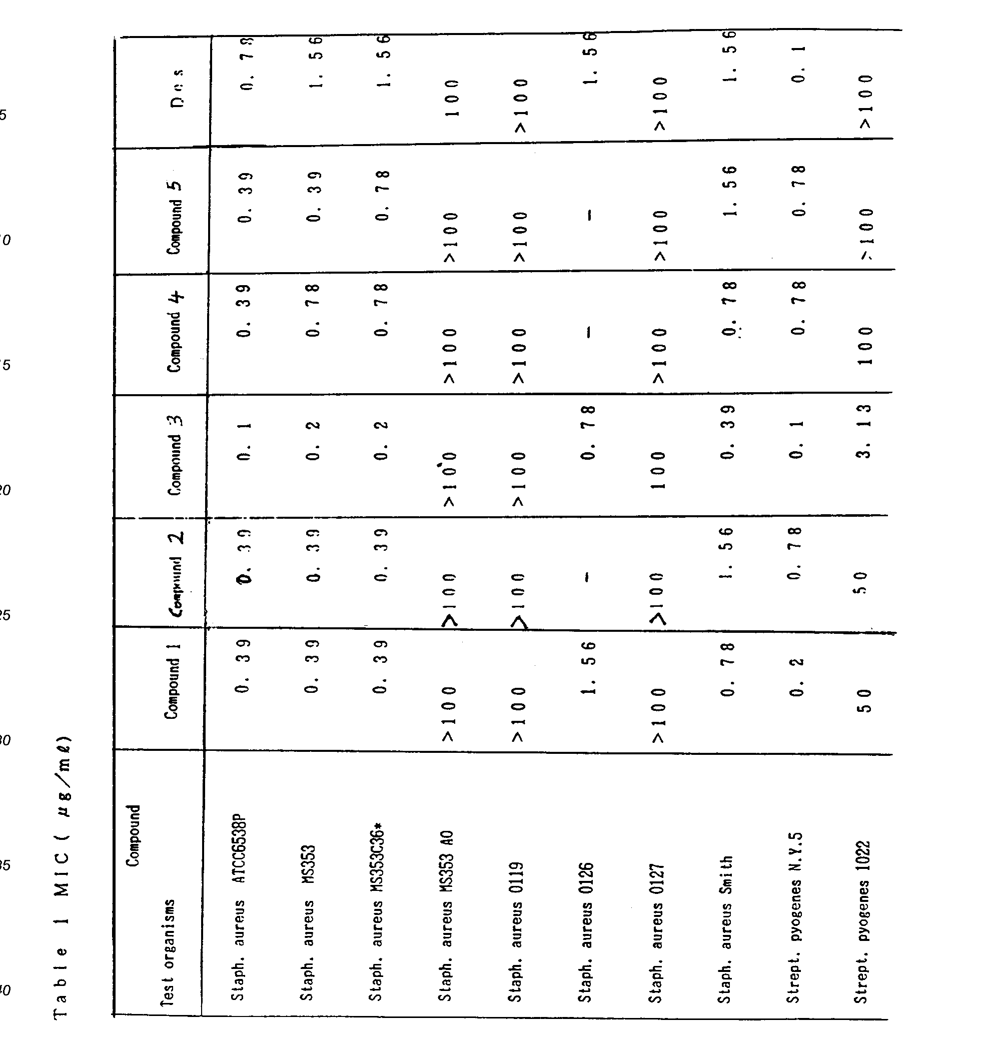

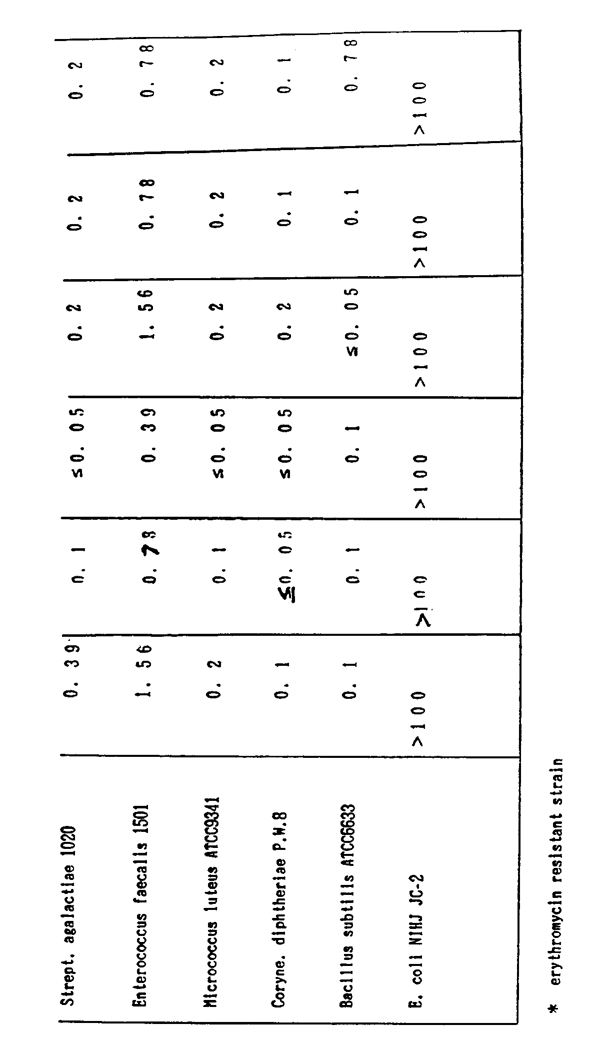

The compounds of the present invention have not only stronger antimicrobial activity against Gram positive bacteria than that of desmycosin (4'-demycarosyl-tylosin) but also strong antimicrobial activity against erythromycin resistant strains. The compounds are therefore useful in the treatment of the human or animal body by therapy, in particular as an antibiotic. The compounds can be formulated as an active ingredient in a pharmaceutical composition also comprising a pharmaceutically acceptable carrier or diluent.

The following Examples illustrate the present invention. In the Example, the silica-gel used in column chromatography is, if not specified, Art 7734 silica-gel (Merck).

### Preparation Example 1

### 2',4'-di-O-acetyl-4"-deoxy-3",4"-di-dehydro-19-deformyldesmycosin:

Tetrabutylammonium fluoride (2.2 g, 1.1 equivalent) was added to 2',4'-di-O-acetyl-4"-O-trifluoromethanesulfonyl-19-deformyldesmycosin (JP-A-58-13596) (6.12 g, 6.38 mmol) dissolved in tetrahydrofuran (60 ml) and stirred at room temperature overnight and at 55°C for 3 hours. After cooling, the reaction mixture was poured into aqueous ammonia and extracted three times with chloroform. An extract was washed with dilute aqueous ammonia and saturated sodium chloride solution, passed through Whatman 1PS filter paper and concentrated in vacuo. The residue was charged onto a column of silica-gel (90 g) packed with benzene and eluted by benzene-acetone (10:1) to obtain white foamy 2',4-di-O-acetyl-4"-deoxy-3",4"-di-dehydro-19-deformyldesmycosin (4.33g, yield: 83.9%).
NMR (PMX-60, CDCl₃, 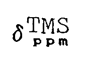
): 1.78(s, 12-Me), 2.05 (s, OAc), 2.33 (s, NMe₂), 3.48 (s, 2"-OMe), 3.52 (s, 3"-OMe), 5.8 (d, H-13), 6.23 (d, H-10), 7.26 (d, H-11)

### Example 2

### 2',4'-di-O-acetyl-4"-deoxy-3"-demethoxy-3"-oxo-19-deformyldesmycosin:

Trifluoroacetic acid (10 ml) was added to 2',4'-di-O-acetyl-4"-deoxy-3",4"-di-dehydro-19-deformyldesmycin (4.33 g, 5.4 m mol) dissolved in a mixture of acetonitrile (50 ml) and water (40 ml) and stirred for overnight. The reaction mixture was alkalized by adding aqueous ammonia, and was extracted three times with chloroform. An extract was washed with saturated sodium chloride solution, passed through Whatman 1PS filter paper and concentrated in vacuo. The residue was charged onto a column of silica-gel (90g) packed with benzene and eluted by benzene-acetone (15 : 1), (9 : 1) and (4 : 1) in this order to obtain white foamy 2',4'-di-O-acetyl-4"-deoxy-3"-demethoxy-3"-oxo-19-deformyldesmycosin (2.73 g, yield: 63.6%).
NMR (PMX-60, CDCl₃, 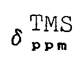
): 1.80 (s, 12-Me), 2.03 (s, OAc), 2.35 (s, NMe₂), 3.52 (s, 2"-OMe), 5.85 (d, H-13), 6.25 (d, H-10), 7.23 (d, H-11)

### Example 3

### 4"-deoxy-3"-demethoxy-3"-oxo-19-deformyldesmycosin:

2',4'-di-O-acety-4"-deoxy-3"-demethoxy-3"-oxo-19-deformyldesmycosin (2.73 g) was dissolved in methanol (30 ml) and stirred at 55°C overnight. The reaction mixture was concentrated in vacuo. The residue was charged onto a column of silica-gel (45 g) packed with chloroform and eluted by chloroform-methanol (70 : 1), (50 : 1) and (30 : 1) in this order to obtain white foamy 4"-deoxy-3"-demethoxy-3"-oxo-19-deformyldesmycosin (1.95 g, yield: 80.1%).
NMR (FX-100, CDCl₃, 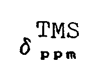
): 1.81 (s, 12-Me), 2.51 ( s, NMe₂), 3.56 (s, 2"-OMe), 4.29 (d, H-1', J=7.57 Hz), 4.39 (d, H-1", J=7.57 Hz), 5.0 (br. t, H-15), 5.87(d, H-13), 6.27 (d, H-10), 7,26 (d, H=11)

### Preparation Example 4

### 2'-O-acetyl-4',4"-di-deoxy-3",4"-di-dehydro-19-deformyldesmycosin:

Tetrabutylammonium fluoride (1.14 g, 1.1 equivalent) was added to 2'-O-acetyl-4'-deoxy-4"-O-trifluoromethanesulfonyl-19-deformyldesmycosin (3.0 g, 3.3 m mol) dissolved in tetrahydrofuran (60 ml) and stirred at room temperature overnight and at 55°C for 3 hours. After cooling the reaction mixture was poured into aqueous ammonium and extracted three times with chloroform. An extract was washed with diluted aqueous ammonia and saturated sodium chloride solution, passed through Whatman 1PS filter paper and concentrated in vacuo. The residue was charged onto a column of silica-gel (50 g) packed with benzene and eluted by benzene-acetone (20 : 1) and (10 : 1) to obtain white foamy 2'-O-acetyl-4',4"-di-deoxy-3",4"-di-dehydro-19-deformyldesmycosin (2.06 g, yield: 83.9%).
NMR (PMX-60, CDCl₃, 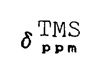
): 1.78 (s, 12-Me), 2.03 (s, OAc), 2.25 (s, NMe₂), 3.45 (s, 2"-OMe), 3.52 (s, 3"-OMe), 4.27 (d, H-1'), 4.58 (d, H-1"), 4.6 (d, H-4"), 5.8 (d, H-13), 6.25 (d, H-10), 7.3 (d, H-11)

### Example 5

### 2'-O-acetyl-4',4"-di-deoxy-3"-demethoxy-3"-oxo-19-deformyldesmycosin:

Trifluoroacetic acid (5 ml) was added to 2'-O-acetyl-4',4"-di-deoxy-3",4"-di-dehydro-19-deformyldesmycosin (2.06 g, 2.74 m mol) dissolved in a mixture of acetonitrile (25 ml) and water (20 ml) under ice-cooling and stirred overnight. The reaction mixture was alkalized by adding aqueous ammonia and was extracted three times with chloroform. An extract was washed with saturated sodium chloride solution, passed through Whatman 1PS filter paper and concentrated in vacuo. The residue was charged onto a column of silica-gel (45 g) packed with benzene and eluted by benzene-acetone (5 : 1) and (3 : 1) in this order to obtain white foamy 2'-O-acetyl-4',4"-di-deoxy-3"-demethoxy-3"-oxo-19-deformyldesmycosin (1.08 g, yield: 32.7%).
NMR (PMX-60, CDCl₃, 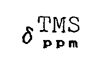
): 1.80 (s, 12-Me), 2.05 (s, OAc), 2.23 (s NMe₂), 3.53 (s, 2"-OMe), 5.83 (d, H-13), 6.25 (d, H-10), 7.26 (d, H-11)

### Example 6

### 4',4"-di-deoxy-3",4"-di-dehydro-19-deformyldesmycosin:

2'-O-acetyl-4',4"-di-deoxy-3",4"-di-dehydro-19-deformyldesmycosin (250 mg) was dissolved in methanol (5 ml) and stirred at 55 °C overnight. The reaction mixture was concentrated in vacuo. The residue was charged onto a column of silica-gel (7 g, Merck, Art 9385) packed with chloroform and eluted by chloroform-methanol (60 : 1) and (40 : 1) in this order to obtain white foamy 4',4"-di-deoxy-3",4"-di-dehydro-19-deformyldesmycosin (180 mg, yield: 76.9%).
NMR (FX-100, CDCl₃, 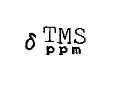
): 1.79(s, 12-Me), 2.27 ( s, NM₂), 3.57 (s, 2"-OMe), 4.24 (d, H-1'), 4.58 (d, H-1"), 4.96 (br. t, H-15), 5.85(d, H-13), 6.30 (d, H-10), 7.29 (d, H-11)
MS(CI, isoBu, m/z): 710(MH+)

### Example 7

### 4',4"-di-deoxy-3"-demethoxy-3"-oxo-19-deformyldesmycosin:

2'-O-acetyl-4',4"-di-deoxy-3"-demethoxy-3"-oxo-19-deformyldesmycosin (150 mg) was dissolved in methanol (2 ml) and stirred at 55 °C overnight. The reaction mixture was concentrated in vacuo. The residue was charged onto a column of silica-gel (6 g) packed with chloroform and eluted by chloroform-methanol (50 : 1) and (40 : 1) in this order to obtain white foamy 4',4"-di-deoxy-3"-demethoxy-3"-oxo-19-deformyldesmycosin (117 mg, yield: 84.2%).
NMR (FX-100, CDCl₃, 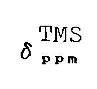
): 1.80 (s, 12-Me), 2.28 ( s, NMe₂), 3.56 (s, 2"-OMe), 4.24 (d, H-1', J=7.32 Hz), 4.39 (d, H 1", J=7.57 Hz), 4.94 (br. t, H-15), 5.85 (d, H-13), 6.32 (d, H-10), 7.30 (d, H-11)
MS (CI, isoBu, m/z): 696 (MH ⁺ )

### Example 8

### 4"-deoxy-3"-demethoxy-3"-methylene-19-deformyldesmycosin:

A hexane solution (3 ml) of butyl lithium (1.5 M, 4.0 eq.) was added dropwise under an argon gas atmosphere to methyl triphenylphosphonium bromide (1.61 g, 4.0 equivalents) suspended in dry tetrahydrofuran (16 ml) and stirred at room temperature for 25 minutes to prepare a methylene triphenylphosphoran solution.

The methylene triphenylphosphoran solution was added dropwise under an argon gas atomosphere to 4"-deoxy-3"-demethoxy-3"-oxo-19-deformyldesmycosin (800 mg, 1.13m mol) dissolved in dry tetrahydrofuran (8 ml) under ice-cooling, stirred for 1.5 hours, and then 0.3 N-HCl was added. The reaction mixture was allowed to stand at room temperature, alkalized by adding aqueous ammonia and extracted with chloroform. An extract was washed with saturated sodium chloride solution, passed through Whatman 1PS filter paper and concentrated in vacuo. The residue was charged onto a column of silica-gel (20 g) packed with chloroform and eluted by chloroform -methanol (150 : 1), (100 : 1), (70 : 1) and (50 : 1) in this order to obtain white foamy 4"-deoxy-3"-demethoxy- 3"-methy lene-19-deformyldesmycosin (190 mg).
NMR (FX-100, CDCl₃, 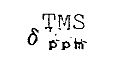
): 1.80 (s, 12-Me), 2.51 ( s, NMe₂), 3.52 (s, 2"-OMe), 4.12 (d, H-1", J=7.57 Hz), 4.29 (d, H-1', J=7.32 Hz), 4.90 and 5.11 (each br. s, 3"-methylene), around 5.0 (br. t, H-15), 5.86 (d, H-13), 6.26 (d, H-10), 7.27 (d, H-11)
MS (CI, isoBu, m/z): 710 (MH ⁺ )

### Example 9

### 4',4"-di-deoxy-3"-demethoxy-3"-methylene-19-deformyldesmycosin:

A hexane solution (0.5 ml) of butyl lithium (1.5 M, 2.5 eq.) was added dropwise under an argon gas atmosphere to methyl triphenylphosphonium bromide (268 mg, 2.5 equivalents) suspended in dry tetrahydrofuran (4 ml) and stirred at room temperature for 10 minutes to prepare a methylene triphenylphosphoran solution.

The methylene triphenylphosphoran solution was added dropwise under an argon gas atomosphere to 2'-O-acetyl-4',4"-di-deoxy-3"-demethoxy-3"-oxo-19-deformyldesmycosin (220 mg, 0.33 m mol) dissolved in dry tetrahydrofuran (2 ml) under ice-cooling, stirred for 1 hour, and then 0.3 N-HCl was added. The reaction mixture was allowed to stand at room temperature, alkalized by adding aqueous ammonia and extracted with chloroform. An extract was washed with saturated sodium chloride solution, passed through Whatman 1PS filter paper and concentrated in vacuo. The residue was charged on a column of silica-gel (10 g) packed with benzene and eluted by benzene-acetone (9 : 1), (7 : 1) and (3 : 1) in this order to obtain white foamy 2'-O-acetyl-4',4"-dideoxy-3"-demethoxy-3"-methylene-19-deformyldesmycosin (107 mg), which was dissolved in methanol (2 ml) and stirred at 55 °C overnight. The reaction mixture was concentrated in vacuo and the residue was charged onto a column of silica-gel (5 g) packed with chloroform and eluted by chloroform-methanol (150 : 1), (100 : 1), (70 : 1) and (50 : 1) in this order to obtain white foamy 4',4"-di-deoxy-3" -demethoxy- 3"-methylene-19-deformyldesmycosin (46 mg, yield: 22%).
NMR (FX-100, CDCl₃, 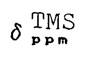
): 1.78 (s, 12-Me), 2.29 ( s, NMe₂), 3.52 (s, 2"-OMe), 4.12 (d, H-1", J=7.3 Hz), 4.29 (d, H- 1', J=7.3 Hz), 4.90 and 5.11 (each br. s, 3"-methylene), around 4.95 (br. t, H-15), around 5.85 (d, H-13), 6.30 (d, H-10), 7.30 (d, H-11)
MS (CI, isoBu, m/z): 694 (MH ⁺ )

### Example 10

### 2',4'-di-O-acetyl-4"-deoxy-3"-demethoxy-3"-methylene 19-deformyldesmycosin:

Acetic anhydride (100 µl, 4 eq.) was added to 4'-deoxy-3"-demethoxy-3"-methylene-19-deformyldesmycosin (190 mg, 0.27 m mol) dissolved in dichloromethane (2 ml) and stirred at room temperature for 1.5 hours. The reaction mixture was diluted with chloroform (5 ml) and aqueous ammonia was added to treat the excess acetic anhydride. The mixture was washed with aqueous ammonia and saturated sodium chloride solution, passed through Whatman 1PS filter paper and concentrated in vacuo to obtain white foamy 2',4'-di-O-acetyl-4"-deoxy-3"-demethoxy-3"-methylene-19-deformyldesmycosin (211 mg, yield: 100%).
NMR (PMX-60, CDCl₃, 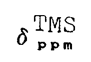
): 1.78 (s, 12-Me), 2.0 (s, OAc x 2), 2.3 (s, NMe₂), 3.45 (s, 2"-OMe), 4.1 (d, H-1"), 4.3 (d, H-1'), 4.8 and 5.05 (each br. s, 3"-methylene), 5.85 (d, H-13), 6.25 (d, H-10), 7.3 (d, H-11)

### Example 11

### 2',4'-di-O-acetyl-4"-deoxy-3"-demethoxy-3"-methylene 3-O-propionyl-19-deformyldesmycosin:

Propionic anhydride (180 µl, 10 eq.) and 4-dimethylamino pyridine (3.5 mg, 0.2 eq.) were added to 2',4'-di-O-acetyl-4"-deoxy-3"-demethoxy-3"-methylene-19-deformyldesmycosin (110 mg, 0.14 m mol) dissolved in pyridine (1.5 ml) and stirred at 55°C for 6.5 hours. The reaction mixture was treated with aqueous ammonia and aqueous sodium chloride (20 vol. excess), and the precipitate was filtered and dissolved in chloroform. The solution was washed with saturated sodium chloride solution, passed through Whatman 1PS filter paper and concentrated in vacuo. The residue was charged onto a column of silica-gel (5 g) packed with benzene and eluted by benzene-acetone (30 : 1), (25 : 1), (20 : 1), (15 : 1), and (10 : 1), in this order to obtain white foamy 2',4'-di-O-acetyl-4"-deoxy-3"-demethoxy-3"-methylene-3-O-propionyl-19-defornyldesmycosin (52.4 mg).
NMR (PMX-60, CDCl₃, 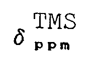
): 1.8(s, 12-Me), 2.05 (s, OAc x 2), 2.3 (q, -CO-CH₂-), 2.35 (3, NMe₂), 3.45 (s, 2"-OMe), 4.1 (d, H-1"), 4.3 (d, H-1'), 4.9 and 5.05 (each br. s, 3"-methylene), 5.15 (d, H-3), 5.8 (d, H-13), 6.2 (d, H-10), 7.25 (d, H-11)

### Example 12

### 4"-deoxy-3"-demethoxy-3"-methylene-3-O-propionyl-19-deformyldesmycosin:

2',4'-di-O-acety-4"-deoxy-3"-demethoxy-3"-methylene-3-O-propionyl-19-deformyldesmycosin (52.4 mg) was dissolved in methanol (2 ml) and stirred at 55°C overnight. The reaction mixture was concentrated in vacuo and the residue was charged onto a column of silica-gel (3.5 g) packed with chloroform and eluted by chloroform-methanol (75 : 1) and (30 : 1) in this order to obtain white foamy 4"-deoxy-3"-demethoxy-3"-methylene-3-O-propionyl-19-deformyldesmycosin (34.5 mg).
NMR (FX-100, CDCl₃, 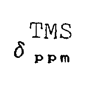
): 1.81 (s, 12-Me), around 2.3 (-CO-CH₂-), 2.54 ( s, NMe₂), 3.49 (s, 2"-OMe), 4.10 (d, H-1", J=7.57 Hz), 4.18 (d, H-1', J=7.57 Hz), 4.89 and 5.09 (each br. s, 3"-methylene), around 4.95 (br. t, H-15), around 5.15 (d, H-3), around 5.9 (d, H-13), 6.21 (d, H-10), 7.33 (d, H-11)
MS (CI, isoBu, m/z): 766 (MH ⁺ )

### Example 13

### 2',4'-di-O-acetyl-4"-deoxy-3"-demethoxy-3"-methylene-3-O-dimethylcarbamoyl-19-deformyldesmycosin:

N,N'-carbonyldiimidazole (105 mg, 5 equivalent) was added to 2',4'-di-O-acetyl-4"-deoxy-3"-demethoxy-3"-methylene-19-deformyldesmycosin (100 mg, 0.13 m mol) dissolved in dichloroethane and stirred at 55 °C overnight. The reaction mixture was poured into water, adjusted by adding diluted hydrochloric acid and extracted three times with chloroform. An extract was washed with diluted aqueous ammonia and saturated sodium chloride solution, passed through Whatman 1PS filter paper and concentrated in vacuo. The residue was dissolved in dichloroethane, bubbled with dimethylamine gas and stirred at room temperature for 1.5 hours. The reaction mixture was concentrated in vacuo and the residue was charged onto a column of silica-gel (5 g) packed with benzene and eluted by benzene-acetone (20 : 1), (15 : 1) (10 : 1) and (5 : 1) to obtain white foamy 2',4'-di-O-acetyl-4"-deoxy-3"-demethoxy-3"-methylene-3-O-dimethylcarbamoyl-19-deformyldesmycosin (50.2 mg).
NMR (PMX-60, CDCl₃, 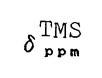
): 1.8 (s, 12-Me), 2.05 (s, OAc x 2) 2.35 (s, 3'-NMe₂), 2.9 (s, -CO-NMe₂), 3.45 (s, 2"-OMe), 4.1 (d, H-1"), 4.3 (d, H-1'), 4.9 and 5.05 (each br. s, 3"-methylene), 5.8 (d, H-13), 6.2 (d, H-10), 7.25 (d, H-11)

### Example 14

### 4"-deoxy-3"-demethoxy-3"-methylene-3-O-dimethylcarbamoyl 19-deformyldesmycosin:

2',4'-di-O-acetyl-4"-deoxy-3"-demethoxy-3"-methylene-3-O-dimethylcarbamoyl-19-deformyldesmycosin (50.2 mg) was dissolved in methanol (2 ml) and stirred at 55 °C overnight. The reaction mixture was concentrated in vacuo and the residue was charged onto a column of silica-gel (3.5 g) packed with chloroform and eluted by chloroform-methanol (75 : 1) and (30 : 1) in this order to obtain white foamy 4"-deoxy-3"-demethoxy-3"-methylene-3-O-methylcarbamoyl-19-deformyldesmycosin (26.4 mg).
NMR (FX-100, CDCl₃, 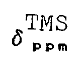
): 1.81 (s, 12-Me), 2.56 (s, 3'-NMe₂), 2.92 (s, -CO-NMe₂), 3.49 (s, 2"-OMe), 4.11 (d, H-1", J=7.32 Hz), 4.22 (d, H-1', J=7.08 Hz), 4.9 and 5.09 (each br. s, 3"-methylene), around 5.1 (d, H-3), around 5.9 (d, H-13), 6.14 (d, H-10), 7.34 (d, H-11) MS (CI, isoBu, m/z): 781 (MH ⁺ ), 692

### Example 15

### 4'4"-di-deoxy-3"-demethoxy-3"-methylene-3-O-(thiophene-2-acetyl)-19-deformyldesmycosin:

Thiophene-2-acetate (1.83 g, 10 eq.), N,N'-dicyclohexylcarbodiimide (1.33 g, 5 eq.) and 4-dimethylamino pyridine (158 mg, 1 eq.) were added to 2'-O-acetyl-4',4"-di-deoxy-3"-demethoxy-3"-methylene-19-deformyldesmycosin (952 mg) dissolved in dichloromethane (25 ml) and stirred at room temperature overnight. The precipitate was filtered and the filtered liquid was washed with aqueous ammonia and saturated sodium chloride solution, passed through Whatman 1PS filter paper and concentrated in vacuo. The residue was charged on a column of silica-gel (23 g) packed with benzene and eluted by benzene-acetone (20 : 1), (15 : 1) and (10 : 1) in this order to obtain white foamy 2'-O-acetyl-4',4"-di-deoxy-3"- demethoxy-3"-methylene-3-O-(thiophene-2-acetyl)-19-deformyldesmycosin (733 mg), which was dissolved in methanol (7 ml) and stirred at 55°C overnight. The reaction mixture was concentrated in vacuo and the residue was charged onto a column of silica-gel (10 g) packed with chloroform and eluted by chloroformmethanol (120 : 1) to obtain white foamy 4',4"-di-deoxy-3"-demethoxy-3"-methylene-3-O-(thiophene-2-acetyl)-19-deformyldesmycosin (612 mg).
NMR (FX-100, CDCl₃, 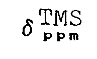
): 1.80 (s 12-Me), 2.27 ( s, NMe₂), 3.50 (s, 2"-OMe), 3.81 (s -CH₂-thiophene), 3.97 (d, H-1', J=7.32 Hz), 4.10 (d, H-1", J=7.57 Hz), 4.89 and 5.11 (each br. s, 3"-methylene), 4.9 (br. t, H-15) 5.15 (d, H-3), 5.85 (d, H-13), 6.27 (d, H-10), 6.93 and 7.12 (m, thiophene), 7.34 (d, H-11)
MS (CI, isoBu, m/z): 818 (MH ⁺ )

## Claims (Claims for the following Contracting State(s): DE, FR, IT)

1. A desmycosin derivative of the formula (1): 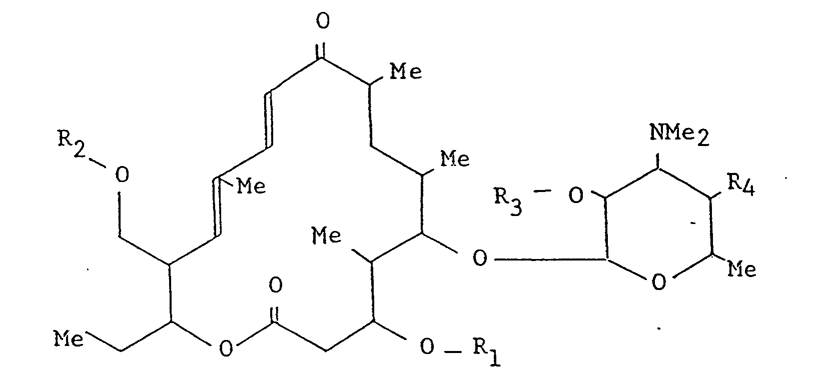 wherein R₁ is hydrogen, C₂-C₅ alkanoyl, unsubstituted benzoyl, unsubstituted phenyl-C₂-C₅ alkanoyl, thienylacetyl or a group of the formula : 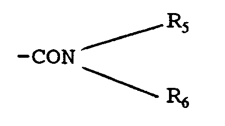 wherein R₅ is C₁-C₄ alkyl, unsubstituted phenyl or unsubstituted phenyl-C₁-C₄ alkyl and R₆ is hydrogen or C₁-C₄ alkyl, R₂ is a 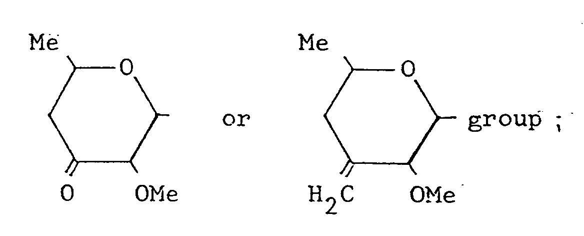 R₃ is hydrogen or a protective group for hydroxyl; and R₄ is hydrogen, hydroxyl or protected hydroxyl; and pharmacologically acceptable salts thereof.

2. A compound according to claim 1, wherein R₃ is hydrogen and R₄ is hydroxyl.

3. A compound according to claim 2, which is:
4"-deoxy-3"-demethoxy-3"-oxo-19-deformyldesmycosin,
4"-deoxy-3"-demethoxy-3"-methylene-19-deformyldesmycosin,
4"-deoxy-3"-demethoxy-3"-methylene-3-O-dimethylcarbamoyl-19-deformyldesmycosin, or
4"-deoxy-3"-demethoxy-3"-methylene-3-O-propiony-19-deformyldesmycosin; or
a pharmacologically acceptable salt thereof.

4. A compound according to claim 1, wherein R₃ or R₄ is hydrogen.

5. A compound according to claim 4, which is:
4',4"-di-deoxy-3"-demethoxy-3"-oxo-19-deformyldesmycosin,
4',4"-di-deoxy-3"-demethoxy-3"-methylene-19-deformyl-desmycosin, or
4',4"-di-deoxy-3"-demethoxy-3"-methylene-3-O-(thiophene-2-acetyl)-19-deformyldesmycosin, or
a pharmacologically acceptable salt thereof.

6. A process for the preparation of a desmycosin derivative of formula (1) as defined in claim 1 or a pharmacologically acceptable salt thereof, which process comprises
(i) effecting 3",4"-dehydroxylation of the mycinose moiety of 19-deformyl- or 19-deformyl-4'-deoxy-desmycosin wherein the 2'- and, in the case of 19-deformyldesmycosin, 4'-hydroxy groups are protected to obtain thereby a desmycosin derivative of formula (1'): 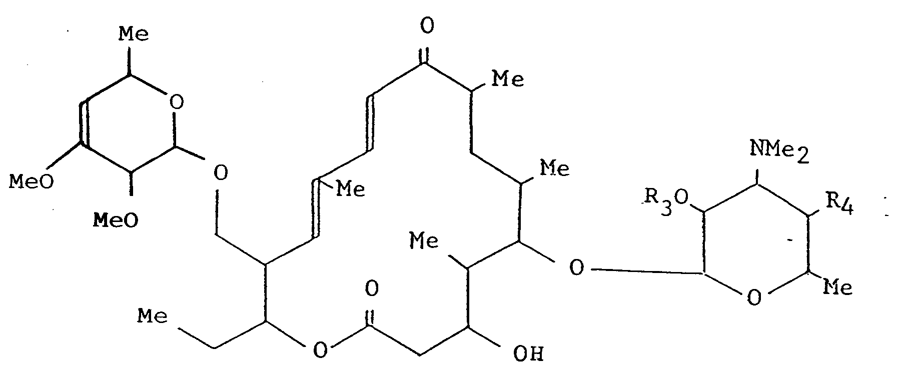 wherein R₃ is a hydroxy-protecting group and R₄ is hydrogen or a protected hydroxy group;
(ii) converting the said desmycosin derivative of formula (1') into a desmycosin derivative of formula (1);
(iii) optionally, removing the or each hydroxy-protecting group; and
(iv) optionally, converting the desmycosin derivative of formula (1) into a pharmacologically acceptable salt thereof.

7. A process according to claim 6, wherein step (i) is effected by treating a said protected 19-deformyl- or 19-deformyl-4'-deoxy-desmycosin bearing a trifluoromethanesulfonyl substituent at the 4"-position with tetrabutylammonium fluoride.

8. A pharmaceutical composition comprising a pharmaceutically acceptable carrier or diluent and, as active ingredient, a desmycosin derivative of formula (1) as defined in any one of claims 1 to 5 or a pharmacologically acceptable salt thereof.

## Claims (Claims for the following Contracting State(s): ES)

1. A process for the preparation of a desmycosin derivative of formula (1): 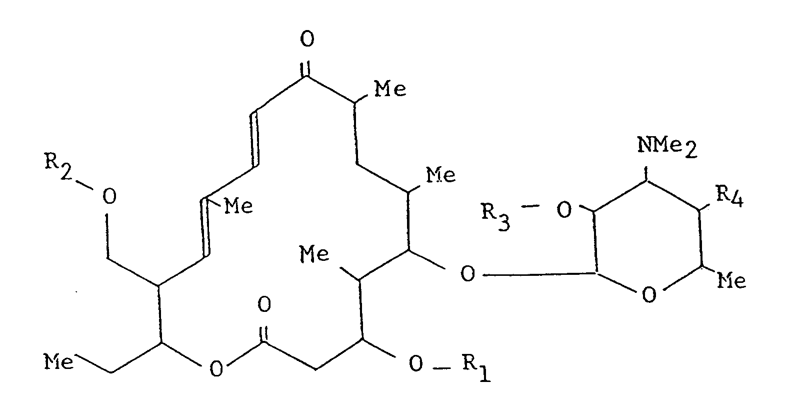 wherein R₁ is hydrogen, C₂-C₅ alkanoyl, unsubstituted benzoyl, unsubstituted phenyl-C₂-C₅ alkanoyl, thienylacetyl or a group of the formula : 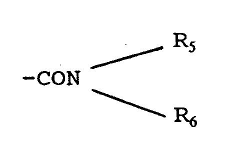 wherein R₅ is C₁-C₄ alkyl, unsubstituted phenyl or unsubstituted phenyl-C₁-C₄ alkyl and R₆ is hydrogen or C₁-C₄ alkyl, R₂ is a 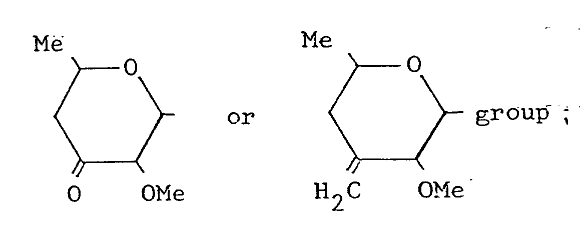 R₃ is hydrogen or a protective group for hydroxyl; and R₄ is hydrogen, hydroxyl or protected hydroxyl; or a pharmacologically acceptable salt thereof, which process comprises
(i) effecting 3",4"-dehydroxylation of the mycinose moiety of 19-deformyl- or 19-deformyl-4'-deoxy-desmycosin wherein the 2'- and, in the case of 19-deformyldesmycosin, 4'-hydroxy groups are protected to obtain thereby a desmycosin derivative of formula (1'): 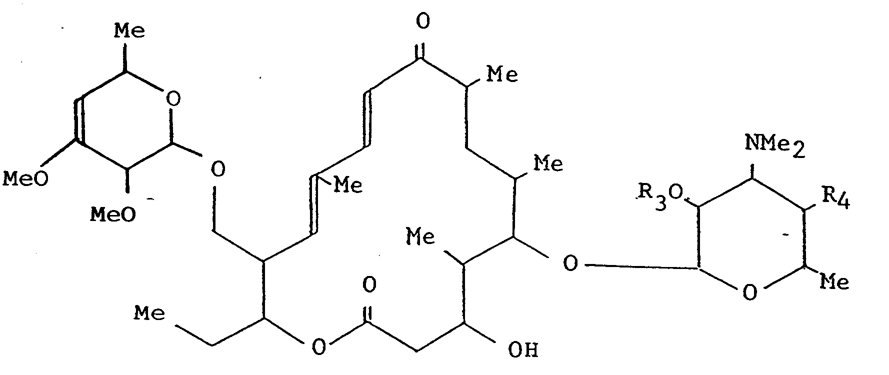 wherein R₃ is a hydroxy-protecting group and R₄ is hydrogen or a protected hydroxy group;
(ii) converting the said desmycosin derivative of formula (1') into a desmycosin derivative of formula (1);
(iii) optionally, removing the or each hydroxy-protecting group; and
(iv) optionally, converting the desmycosin derivative of formula (1) into a pharmacologically acceptable salt thereof.

2. A process according to claim 1, wherein step (i) is effected by treating a said protected 19-deformyl- or 19-deformyl-4'-deoxy-desmycosin bearing a trifluoromethanesulfonyl substituent at the 4"-position with tetrabutylammonium fluoride.

3. A process according to claim 1 or 2, wherein R₃ is hydrogen and R₄ is hydroxyl.

4. A process according to claim 3, wherein the compound of formula (1) is:
4"-deoxy-3"-demethoxy-3"-oxo-19-deformyldesmycosin,
4"-deoxy-3"-demethoxy-3"-methylene-19-deformyldesmycosin,
4"-deoxy-3"-demethoxy-3"-methylene-3-O-dimethylcarbamoyl-19-deformyldesmycosin, or
4"-deoxy-3"-demethoxy-3"-methylene-3-O-propionyl-19-deformyldesmycosin; or
a pharmacologically acceptable salt thereof.

5. A process according to claim 1 to 2, wherein R₃ or R₄ is hydrogen.

6. A process according to claim 5, wherein the compound of formula (1) is:
4',4"-di-deoxy-3"-demethoxy-3"-oxo-19-deformyldesmycosin,
4',4"-di-deoxy-3"-demethoxy-3"-methylene-19-deformyl-desmycosin, or
4',4"-di-deoxy-3"-demethoxy-3"-methylene-3-O-(thiophene-2-acetyl)-19-deformyldesmycosin, or
a pharmacologically acceptable salt thereof.

7. A process for preparing a pharmaceutical composition, which comprises mixing together a pharmaceutically acceptable carrier or diluent and, as active ingredient, a desmycosin derivative of formula (1) as defined in any one of claims 1 or 3 to 6 or a pharmacologically acceptable salt thereof.

## Patentansprüche (Patentansprüche für folgende(n) Vertragsstaat(en): DE, FR, IT)

1. Desmycosinderivat der Formel (1): 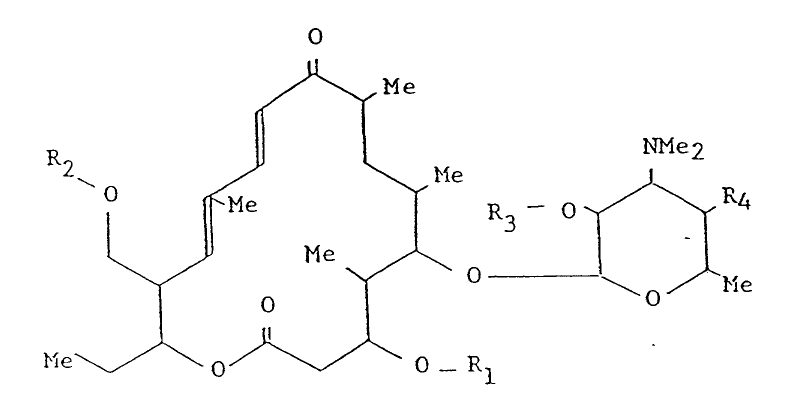 worin R₁ Wasserstoff, C₂-C₅-Alkanoyl, unsubstituiertes Benzoyl, unsubstituiertes Phenyl-C₂-C₅-alkanoyl, Thienylacetyl oder eine Gruppe der Formel: 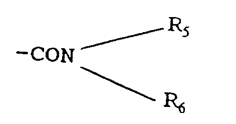 worin R₅ für C₁-C₄-Alkyl, unsubstituiertes Phenyl oder unsubstituiertes Phenyl-C₁-C₄-alkyl und R₆ für Wasserstoff oder C₁-C₄-Alkyl stehen, bedeutet, R₂ eine 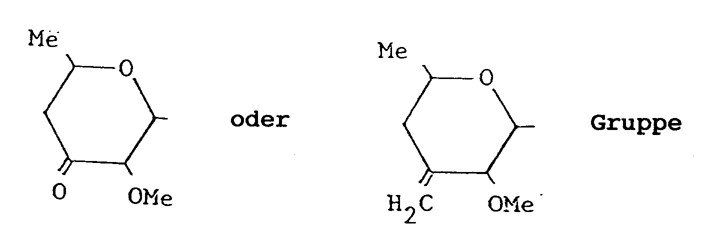 bedeutet,
R₃ Wasserstoff oder eine Schutzgruppe für Hydroxyl bedeutet und R₄ Wasserstoff, Hydroxyl oder geschütztes Hydroxyl bedeutet, und die pharmakologisch annehmbaren Salze davon.

2. Verbindung nach Anspruch 1, dadurch **gekennzeichnet,** daß R₃ Wasserstoff und R₄ Hydroxyl bedeuten.

3. Verbindung nach Anspruch 2, nämlich:
4''-Deoxy-3''-demethoxy-3''-oxo-19-deformyl-desmycosin,
4''-Deoxy-3''-demethoxy-3''-methylen-19-deformyldesmycosin,
4''-Deoxy-3''-demethoxy-3''-methylen-3-O-dimethylcarbamoyl-19-deformyldesmycosin, oder
4''-Deoxy-3''-demethoxy-3''-methylen-3-O-propionyl-19-deformyldesmycosin, oder
ein pharmakologisch annehmbares Salz davon.

4. Verbindung nach Anspruch 1, dadurch **gekennzeichnet,** daß R₃ oder R₄ Wasserstoff bedeuten.

5. Verbindung nach Anspruch 4, nämlich:
4',4''-Di-deoxy-3''-demethoxy-3''-oxo-19-deformyldesmycosin,
4',4''-Di-deoxy-3''-demethoxy-3''-methylen-19-deformyldesmycosin, oder
4',4''-Di-deoxy-3''-demethoxy-3''-methylen-3-O-(thiophen-2-acetyl)-19-deformyldesmycosin oder
ein pharmakologisch annehmbares Salz davon.

6. Verfahren zur Herstellung eines Desmycosinderivats der Formel (1) nach Anspruch 1 oder eines pharmakologisch annehmbaren Salzes davon, dadurch **gekennzeichnet,** daß
(i) eine 3'',4''-Dehydroxylierung der Mycinosegruppe des 19-Deformyl oder 19-Deformyl-4'-deoxydesmycosin, worin die 2'- und im Falle von 19-Deformyldesmycosin, die 4'-Hydroxygruppen geschützt sind, durchgeführt wird, wobei ein Desmycosinderivat der Formel (1') 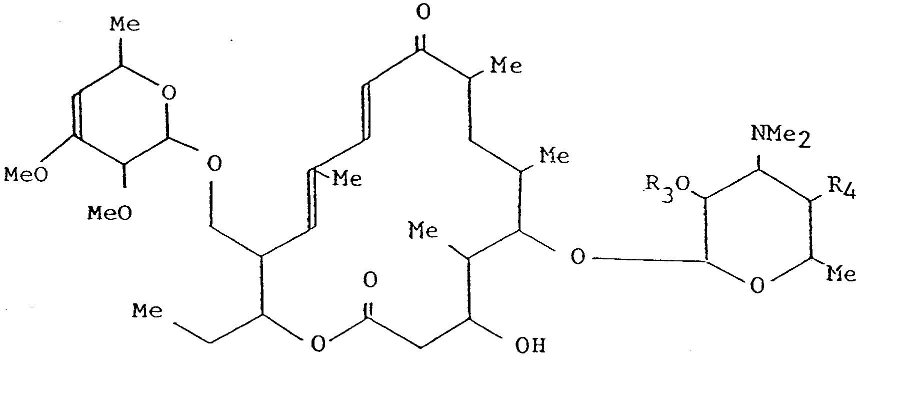 erhalten wird,
worin R₃ eine Hydroxy-Schutzgruppe bedeutet und R₄ Wasserstoff oder eine geschützte Hydroxygruppe bedeutet;
(ii) das Desmycosinderivat der Formel (1') in ein Desmycosinderivat der Formel (1) überführt wird;
(iii) gegebenenfalls die oder jede Hydroxy-Schutzgruppe entfernt wird bzw. werden; und
(iv) gegebenenfalls das Desmycosinderivat der Formel (1) in ein pharmakologisch annehmbares Salz davon überführt wird.

7. Verfahren nach Anspruch 6, dadurch **gekennzeichnet,** daß die Stufe (i) durch Behandlung des geschützten 19-Deformyl- oder 19-Deformyl-4'-deoxydesmycosin, das einen Trifluormethansulfonyl-Substituent in der 4''-Stellung trägt, mit Tetrabutylammoniumfluorid durchgeführt wird.

8. Pharmazeutisches Präparat, dadurch **gekennzeichnet,** daß es einen pharmazeutisch annehmbaren Träger oder ein Verdünnungsmittel und als aktiven Bestandteil ein Desmycosinderivat der Formel (1), wie es in einem der Ansprüche 1 bis 5 definiert wurde oder ein pharmakologisch annehmbares Salz davon enthält.

## Patentansprüche (Patentansprüche für folgende(n) Vertragsstaat(en): ES)

1. Verfahren zur Herstellung eines Desmycosinderivats der Formel (1) 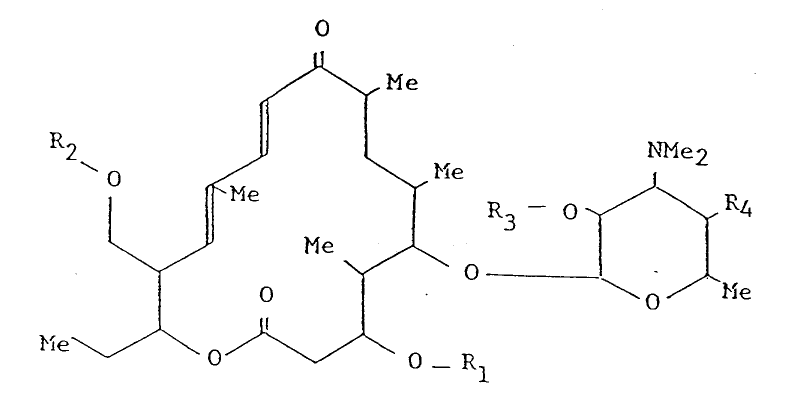 worin R₁ Wasserstoff, C₂-C₅-Alkanoyl, unsubstituiertes Benzoyl, unsubstituiertes Phenyl-C₂-C₅-alkanoyl, Thienylacetyl oder eine Gruppe der Formel: 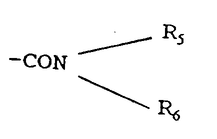 worin R₅ für C₁-C₄-Alkyl, unsubstituiertes Phenyl oder unsubstituiertes Phenyl-C₁-C₄-alkyl und R₆ für Wasserstoff oder C₁-C₄-Alkyl stehen, bedeutet, R₂ eine 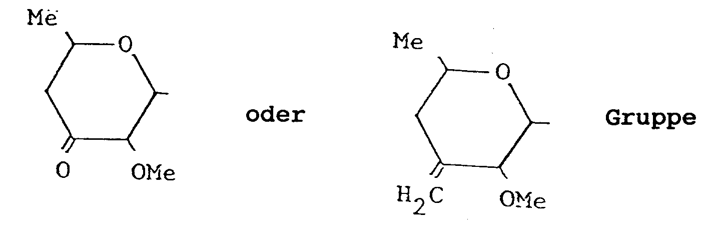 bedeutet,
R₃ Wasserstoff oder eine Schutzgruppe für Hydroxyl bedeutet und R₄ Wasserstoff, Hydroxyl oder geschütztes Hydroxyl bedeutet, oder eines pharmakologisch annehmbaren Salzes davon, dadurch **gekennzeichnet,** daß
(i) eine 3'',4''-Dehydroxylierung der Mycinosegruppe des 19-Deformyl oder 19-Deformyl-4'-deoxydesmycosin, worin die 2'- und im Falle von 19-Deformyldesmycosin, die 4'-Hydroxygruppen geschützt sind, durchgeführt wird, wobei ein Desmycosinderivat der Formel (1') 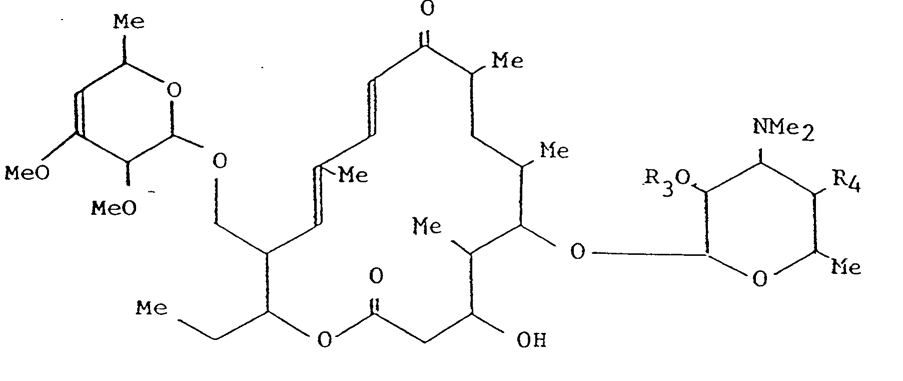 erhalten wird,
worin R₃ eine Hydroxy-Schutzgruppe bedeutet und R₄ Wasserstoff oder eine geschützte Hydroxygruppe bedeutet;
(ii) das Desmycosinderivat der Formel (1') in ein Desmycosinderivat der Formel (1) überführt wird;
(iii) gegebenenfalls die oder jede Hydroxy-Schutzgruppe entfernt wird bzw. werden; und
(iv) gegebenenfalls das Desmycosinderivat der Formel (1) in ein pharmakologisch annehmbares Salz davon überführt wird.

2. Verfahren nach Anspruch 1, dadurch **gekennzeichnet,** daß die Stufe (i) durch Behandlung des geschützten 19-Deformyl- oder 19-Deformyl-4'-deoxydesmycosin, das einen Trifluormethansulfonyl-Substituenten in der 4''-Stellung trägt, mit Tetrabutylammoniumfluorid durchgeführt wird.

3. Verfahren nach Anspruch 1 oder 2, dadurch **gekennzeichnet,** daß R₃ Wasserstoff und R₄ Hydroxyl bedeuten.

4. Verfahren nach Anspruch 3, dadurch **gekennzeichnet,** daß die Verbindung der Formel (1)
4''-Deoxy-3''-demethoxy-3''-oxo-19-deformyl-desmycosin,
4''-Deoxy-3''-demethoxy-3''-methylen-19-deformyldesmycosin,
4''-Deoxy-3''-demethoxy-3''-methylen-3-O-dimethylcarbamoyl-19-deformyldesmycosin, oder
4''-Deoxy-3''-demethoxy-3''-methylen-3-O-propionyl-19-deformyldesmycosin, oder
ein pharmakologisch annehmbares Salz davon ist.

5. Verfahren nach Anspruch 1 oder 2, dadurch **gekennzeichnet,** daß R₃ oder R₄ Wasserstoff bedeutet.

6. Verfahren nach Anspruch 5, dadurch **gekennzeichnet,** daß die Verbindung der Formel (1)
4',4''-Di-deoxy-3''-demethoxy-3''-oxo-19-deformyldesmycosin,
4',4''-Di-deoxy-3''-demethoxy-3''-methylen-19-deformyldesmycosin, oder
4',4''-Di-deoxy-3''-demethoxy-3''-methylen-3-O-(thiophen-2-acetyl)-19-deformyldesmycosin oder
ein pharmakologisch annehmbares Salz davon ist.

7. Verfahren zur Herstellung eines pharmazeutischen Präparats, dadurch **gekennzeichnet,** daß ein pharmazeutisch annehmbarer Träger oder ein Verdünnungsmittel und als aktiver Bestandteil ein Desmycosinderivat der Formel (1), wie es in einem der Ansprüche 1 oder 3 bis 6 definiert ist, oder ein pharmakologisch annehmbares Salz davon zusammen vermischt werden.

## Revendications (Revendications pour l'(les) Etat(s) contractant(s) suivant(s): DE, FR, IT)

1. Dérivé de desmycosine de la formule (1) 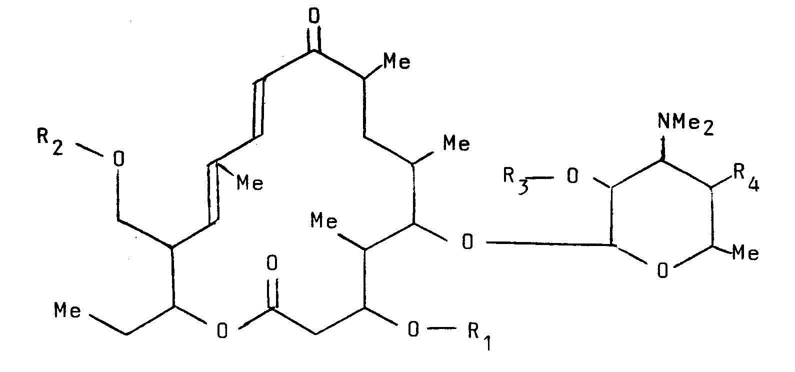 où R₁ est hydrogène, alcanoyle C₂-C₅,benzoyle non substitué, phényle non substitué-alcanoyle C₂-C₅, thiénylacétyle ou un groupe de la formule : 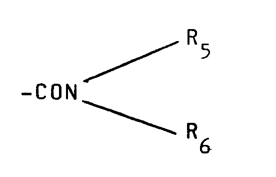 où R₅ est alkyle C₁-C₄, phényle non substitué ou phényle non substitué-alkyle C₁-C₄ et R₆ est hydrogène ou alkyle C₁-C₄, R₂ est un groupe 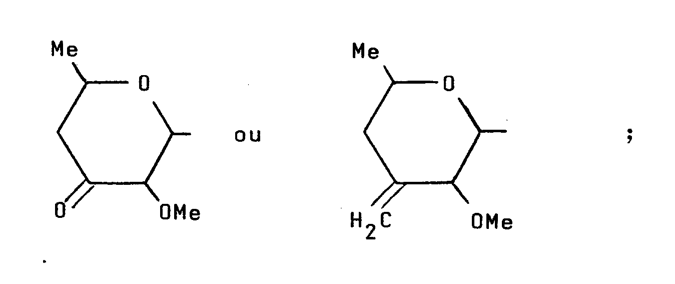 R₃ est hydrogène ou un groupe protecteur pour hydroxyle ; et R₄ est hydrogène, hydroxyle ou un hydroxyle protégé et les sels acceptables en pharmacie.

2. Composé selon la revendication 1, où R₃ est hydrogène et R₄ est hydroxyle.

3. Composé selon la revendication 2, qui est :
4"-désoxy-3"-déméthoxy-3"-oxo-19-déformyldesmycosine,
4"-désoxy-3"-déméthoxy-3"-méthylène-19-déformyldesmycosine,
4"-désoxy-3"-déméthoxy-3"-méthylène-3-O-diméthylcarbamoyl-19-déformyldesmycosine, ou
4"-désoxy-3"-déméthoxy-3"-méthylène-3-O-propionyl-19-déformyldesmycosine ; ou
un sel acceptable en pharmacie.

4. Composé selon la revendication 1, où R₃ ou R₄ est hydrogène.

5. Composé selon la revendication 4, qui est :
4',4"-di-désoxy-3"-déméthoxy-3"-oxo-19-déformyldesmycosine,
4',4"-di-désoxy-3"-déméthoxy-3"-méthylène-19-déformyldesmycosine, ou
4',4"-di-désoxy-3"-déméthoxy-3"-méthylène-3-O-(thiophène-2-acétyl)-19-déformyldesmycosine, ou
un sel acceptable en pharmacie.

6. Procédé de préparation d'un dérivé de desmycosine de formule (1), tel que défini à la revendication 1, ou son sel acceptable en pharmacie, lequel procédé consiste à :
(i) effectuer la 3",4"-déshydroxylation du fragment de mycinose de la 19-déformyl- ou 19-déformyl-4'-désoxy-desmycosine, où les groupes 2'- et, dans le cas de la 19-déformyldesmycosine, 4'-hydroxy, sont protégés, pour obtenir ainsi un dérivé de desmycosine de la formule (1') : 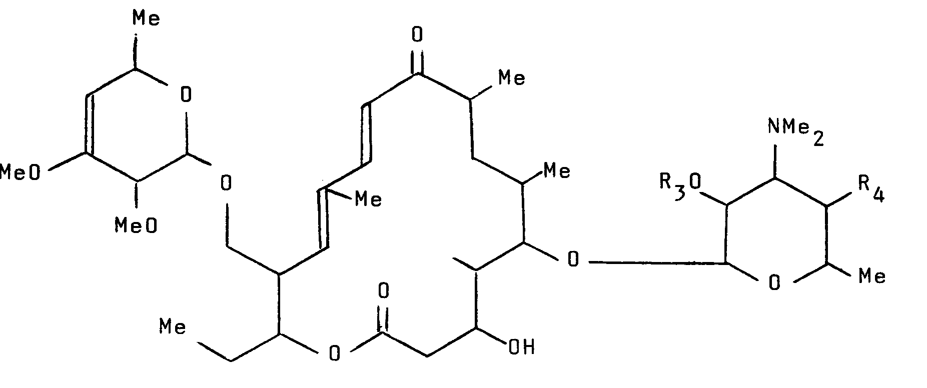 où R₃ est un groupe hydroxy-protecteur et R₄ est hydrogène ou un groupe hydroxy-protégé ;
(ii) convertir ledit dérivé de desmycosine de formule (1') en un dérivé de desmycosine de formule (1) ;
(iii) éliminer facultativement le ou chaque groupe hydroxy-protecteur ; et
(iv) facultativement convertir le dérivé de desmycosine de formule (1) en un sel acceptable en pharmacie.

7. Procédé selon la revendication 6, où l'étape (i) est effectuée par traitement d'une 19-déformyl- ou 19-déformyl-4'-désoxydesmycosine protégée portant un trifluorométhanesulfonyle substituant à la position 4" par du fluorure de tétrabutylammonium.

8. Composition pharmaceutique comprenant un support ou diluant acceptable en pharmacie et, comme ingrédient actif, un dérivé de desmycosine de formule (1),selon l'une quelconque des revendications 1 à 5, ou son sel acceptable en pharmacie.

## Revendications (Revendications pour l'(les) Etat(s) contractant(s) suivant(s): ES)

1. Procédé de préparation d'un dérivé de desmycosine de la formule (1) : 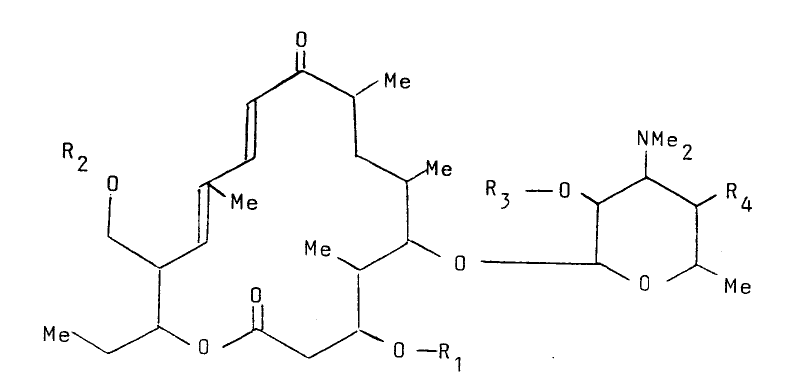 où R₁ est hydrogène, alcanoyle C₂-C₅, benzoyle non substitué, phényle non substitué-alcanoyle C₂-C₅, thiénylacétyle ou un groupe de la formule 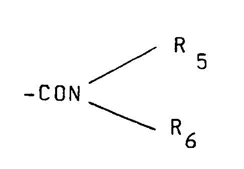 où R₅ est alkyle C₁-C₄, phényle non substitué ou phényle non substitué-alkyle C₁-C₄ et R₆ est hydrogène ou alkyle C₁-C₄, R₂ est un groupe 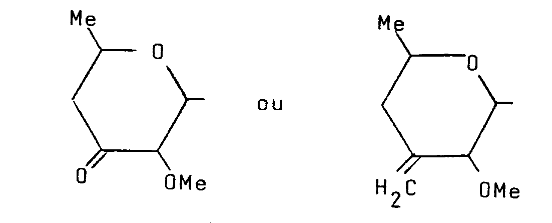 R₃ est hydrogène au un groupe protecteur pour hydroxyle ; et R₄ est hydrogène, hydroxyle ou un hydroxyle protégé et les sels acceptables en pharmacie ; lequel procédé consiste à :
(i) effectuer la 3",4"-déshydroxylation du fragment de mycinose de la 19-déformyl- ou 19-déformyl-4'-désoxy-desmycosine, où les groupes 2'- et, dans le cas de la 19-déformyldesmycosine,4'-hydroxy, sont protégés pour obtenir ainsi un dérivé de desmycosine de la formule (1') : 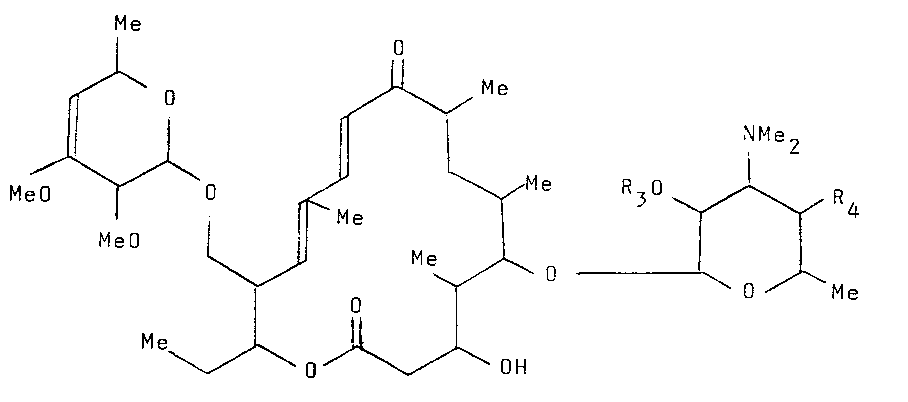 où R₃ est un groupe hydroxy-protecteur et R₄ est hydrogène ou un groupe hydroxy-protégé ;
(ii) convertir ledit dérivé de desmycosine de formule (1') en un dérivé de desmycosine de formule (1) ;
(iii) éliminer facultativement le ou chaque groupe hydroxy-protecteur ; et
(iv) facultativement convertir le dérivé de desmycosine de formule (1) en un sel acceptable en pharmacie.

2. Procédé selon la revendication 1, où l'étape (i) est effectuée par traitement d'une 19-déformyl- ou 19-déformyl-4'-désoxy-desmycosine protégée portant un trifluorométhanesulfonyle substituant à la position 4" par du fluorure de tétrabutylammonium.

3. Procédé selon la revendication 1 ou 2, où R₃ est hydrogène et R₄ est hydroxyle.

4. Procédé selon la revendication 3, où le composé de formule (I) est :
4"-désoxy-3"-déméthoxy-3"-oxo-19-déformyldesmycosine,
4"-désoxy-3"-déméthoxy-3"-méthylène-19-déformyldesmycosine,
4"-désoxy-3"-déméthoxy-3"-méthylène-3-O-diméthylcarbamoyl-19-déformyldesmycosine, ou
4"-désoxy-3"-déméthoxy-3"-méthylène-3-O-propionyl-19-déformyldesmycosine ; ou
un sel acceptable en pharmacie.

5. Procédé selon la revendication 1 ou 2, où R₃ ou R₄ est hydrogène.

6. Procédé selon la revendication 5, où le composé de formule (1) est :
4',4"-di-désoxy-3"-déméthoxy-3"-oxo-19-déformyldesmycosine,
4',4"-di-désoxy-3"-déméthoxy-3"-méthylène-19-déformyldesmycosine, ou
4',4"-di-désoxy-3"-déméthoxy-3"-méthylène-3-O-(thioppène-2-acétyl)-19-déformyldesmycosine, ou
un sel acceptable en pharmacie.

7. Procédé de préparation d'une composition pharmaceutique qui consiste à mélanger un support ou diluant acceptable en pharmacie et, en tant qu'ingrédient actif, un dérivé de desmycosine de la formule (1) selon l'une quelconque des revendications 1 ou 3 à 6 ou un sel acceptable en pharmacie.
